# EUROPEAN PATENT APPLICATION

(11) **EP 1 122 241 A1**
(43) Date of publication of application: **08.08.2001**
(21) Application number: 00200365.5
(22) Date of filing: 03.02.2000
(51) Int. Cl.: C07D 201/08, C07D 201/12

(54) **Process for the preparation of epsilon-caprolactam**

(71) Applicant: DSM N.V., 6411 TE Heerlen (NL)
(72) Inventor: Agterberg, Frank Petrus Willibrord, 6118 EH Nieustadt (NL); Guit, Rudolf Philippus Maria, 6228 GP Maastricht (NL); Offermans, Matthias Robert Jozef, 6374 PM Landgraaf (NL)
(74) Representative: Verhaegen, Ilse Maria M.

(57) **Abstract**

A process for the preparation of ε-caprolactam starting from 6-aminocaproic acid, 6-aminocaproamide, 6-aminocaproic ester, 6-aminocapronitrile, oligomers or polymers of these compounds or mixtures comprising at least two of these compounds, which process is performed in the presence of N-(5-carboxypentyl)-ε-caprolactam and/or derivative thereof in an amount of less than 50 wt.% and more than 0.1 wt.% (based on the total reaction mixture).

## Description

The invention relates to a process for the preparation of ε-caprolactam starting from 6-aminocaproic acid, 6-aminocaproamide, 6-aminocaproic ester, 6-aminocapronitrile, oligomers or polymers of these compounds or mixtures comprising at least two of these compounds.

Such a process is described in JP-A-51039684. This patent publication describes a process to prepare ε-caprolactam from nylon-6 polymer using an adduct of ε-caprolactam and terephtalic acid, isophtalic acid or adipic acid as depolymerisation catalyst.

A disadvantage of this process is that the catalyst has to be produced in advance of the depolymerisation reaction in a separate reaction step. Another disadvantage is that these adducts are process-foreign compounds resulting in ε-caprolactam containing undesired impurities.

The object of the present invention is a process for the preparation of ε-caprolactam in which the above mentioned disadvantages are avoided or at least reduced.

This object is achieved in that the process is performed in the presence of N-(5-carboxypentyl)-ε-caprolactam and/or derivative thereof in an amount of less than 50 wt.% and more than 0.1 wt.% (based on the total reaction mixture).

It has been found that the rate of the reaction of the process according to the invention is significantly higher than the rate of reaction of a process for the preparation of ε-caprolactam starting from the above mentioned compounds performed in the substantial absence of N-(5-carboxypentyl)-ε-caprolactam and/or derivative thereof or performed in the presence of higher amounts of N-(5-carboxypentyl)-ε-caprolactam and/or derivative thereof. Another advantage is that N-(5-carboxypentyl)-ε-caprolactam and/or derivative thereof is a process-own compound so that it is not necessary to remove the N-(5-carboxypentyl)-ε-caprolactam and/or derivative thereof from the ε-caprolactam obtainded in the process according to the invention.

JP-A-45022946 describes a process for the preparation of ε-caprolactam by reacting N-(ω-carbamoylpentyl)-ε-caprolactam with aqueous ammonia at a temperature of 350°C. The compound N-(5-carboxypentyl)-ε-caprolactam itself is known from JP-A-45022946. The use of N-(5-carboxypentyl)-ε-caprolactam or derivative thereof as reaction rate enhancing compound in a process for preparing ε-caprolactam from 6-aminocaproic acid, 6-aminocaproamide, 6-aminocaproic ester, 6-aminocapronitrile, oligomers or polymers of these compounds or mixtures comprising at least two of these compounds is however not disclosed. N-(5-carboxypentyl)-ε-caprolactam is said to be obtained by processing the distillation residue of the caprolacton method in various steps. Moreover, this patent publication teaches away from the present invention in that this publication teaches that the N-(5-carboxypentyl)-ε-caprolactam has first to be converted into N-(5-amidepentyl)-ε-caprolactam before the latter compound is reacted with aqueous ammonia.

With N-(5-carboxypentyl)-ε-caprolactam is meant a compound with the following structural formula:

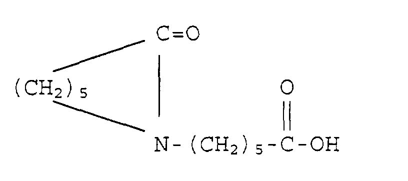

With N-(ω-carbamoylpentyl)-ε-caprolactam is meant a compound with the following structural formula:

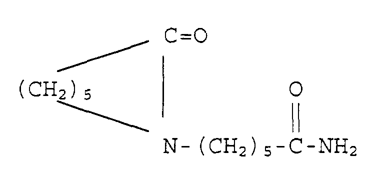

In the present application, the term N-(5-carboxypentyl) ε-caprolactam derivative include compounds with the following structural formula: 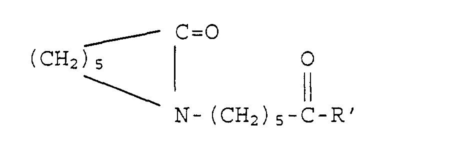 or with the following structural formula: 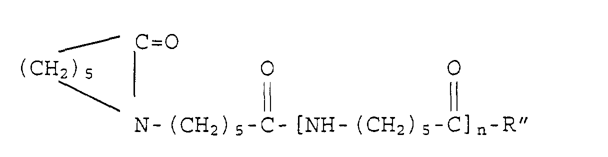 wherein R' is NH₂ or OR¹; n is at least 1; R" is OH, NH2 or OR¹; R¹ is preferably an organic group with 1 to 20 carbon atoms and more preferably with 1 to 6 carbon atoms. The organic group is an alkyl, cycloalkyl, aryl or aralkyl group. More preferably R¹ is an alkyl group. Examples of R¹ groups include methyl, ethyl, propyl, isopropyl, n-butyl, tert-butyl, isobutyl, cyclohexyl, benzyl and phenyl. By preference R¹ is methyl or ethyl.

In case the term N-(5-carboxypentyl)-ε-caprolactam is stated below, it should be read as N-(5-carboxypentyl)-ε-caprolactam and/or derivative thereof.

The N-(5-carboxypentyl)-ε-caprolactam may be added at the beginning of the process according to the present invention. The N-(5-carboxypentyl)-ε-caprolactam may also already be present in a reaction mixture obtained in a previous process step, which also contains at least one of 6-aminocaproic acid, 6-aminocaproamide, 6-aminocaproic ester, 6-aminocapronitrile, oligomers or polymers of these compounds.

Because of the presence of N-(5-carboxypentyl)-ε-caprolactam in the reaction mixture, the reaction rate appears to increase. It has been found that the presence of N-(5-carboxypentyl)-ε-caprolactam in the reaction mixture in an amount higher than 50 wt.% results in a decrease of the reaction rate. The amount of N-(5-carboxypentyl)-ε-caprolactam in the reaction mixture is preferably between 0.1 wt.% and 10 wt.%.

The amount of N-(5-carboxypentyl)-ε-caprolactam can also be kept at and/or brought to the desired value within the above general and preferred ranges by adding or by removing N-(5-carboxypentyl)-ε-caprolactam, for example by purging.

The starting mixture comprising 6-aminocaproic acid, 6-aminocaproate ester, 6-aminocaproamide, 6-aminocapronitrile, oligomers of these compounds and/or polymers of these compounds can be obtained by various processes. For example in US-A-4730040 a process is described in which an aqueous mixture is obtained containing 6-aminocaproic acid and some ε-caprolactam starting from 5-formylvalerate ester. Further in EP-A-729943 a process is described in which an aqueous mixture is obtained containing 6-aminocaproic acid, 6-aminocaproamide and ε-caprolactam also starting from a 5-formylvalerate ester. US-A-5068398 describes a process in which an aqueous mixture is obtained containing 6-aminocaproate ester and some ε-caprolactam starting from a 5-formylvalerate ester. WO-A-9835938 describes a process in which an aqueous mixture is obtained containing ε-caprolactam and 6-aminocaproic acid and/or 6-aminocaproamide starting from 5-formylvaleric acid or from an alkyl 5-formylvalerate in water.

Starting mixtures can also be obtained starting from 6-aminocapronitrile as for example described in WO-A-9837063. Processes starting from 6-aminocapronitrile can yield mixtures comprising 6-aminocaproic acid. Such mixtures can be advantageously used in the process according to the invention. Firstly, the 6-aminocapronitrile is contacted with water under hydrolysis conditions. Secondly, water and ammonia, which is formed in the hydrolysis step, are preferably separated. In a third step the resulting mixture is treated with the process according to the invention.

The process according to the invention can also be applied on aqueous starting mixtures containing 6-aminocapronitrile. Processes to prepare ε-caprolactam starting from 6-aminocapronitrile are for example described in US-A-5495016 and EP-A-659741.

Other examples of starting mixtures which can be used in the process according to the invention are polycaprolactam processing waste, polycaprolactam carpet waste and/or polycaprolactam extraction wash water.

The starting compound or mixture of starting compounds which are obtainable by the above described processes, are preferably contacted with the superheated steam as a liquid, for example as a melt.

Polycaprolactam waste is preferably fed to the reactor as a melt. This feeding may be achieved by using an extruder, gear pump, or other means known in the art.

The process according to the present invention can be applied with particular advantage using an aqueous mixture obtained in a previous process step, which mixture already contains 6-aminocaproic acid, 6-aminocaproamide, 6-aminocaproic ester, 6-aminocapronitrile, oligomers and/or polymers of these compounds. An aqueous mixture containing 6-aminocaproic acid, 6-aminocaproamide, oligomers thereof and ε-caprolactam can with particular advantage be obtained through reductive amination of 5-formylvaleric acid or its ester as for example described in EP-A-729934 or WO-A-9835938.

The 6-aminocaproic acid, 6-aminocaproamide and 6-aminocaproic ester compound respectively are of the following formula: 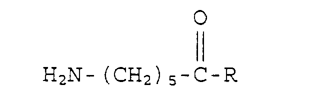 wherein R is OH, NH₂ and OR¹ respectively. R¹ is as defined above.

The 6-aminocapronitrile compound is of the following formula:

H₂N- (CH₂)₅-C≡N

Without limiting ourselves to the following theory, we expect that N-(5-carboxypentyl)-ε-caprolactam may for example be formed from di-(5-carboxypentyl)amine according to the following reaction scheme:

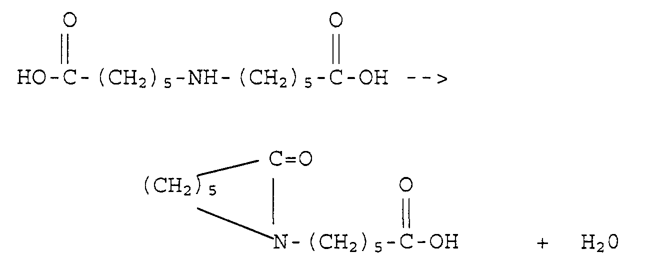

The di-(5-carboxypentyl)amine may for example be formed from 6-aminocaproic acid according to the following reaction scheme: 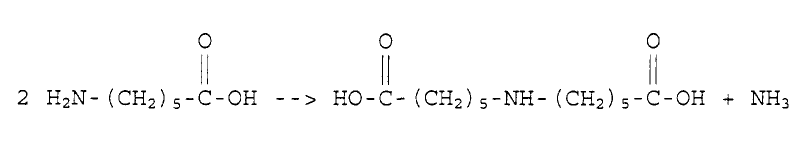 The di-(5-carboxypentyl)amine may for example be formed from 6-aminocaproic acid and a 5-formylvaleric acid ester according to the following reaction scheme:

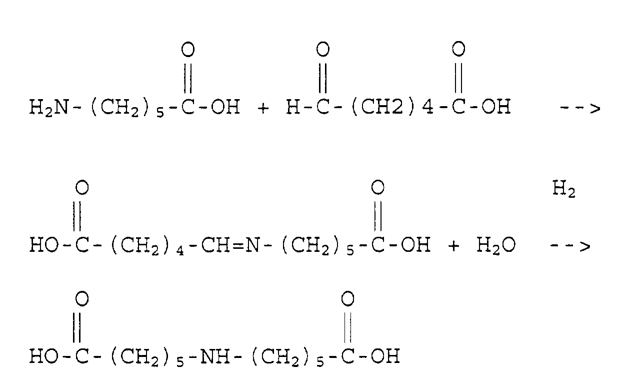

The process according to the invention is performed at a temperature of between about 200 and 400 °C. Preferably, the temperature is between about 270 and 350 °C. More preferably, the temperature is higher than 280 °C because higher selectivities to ε-caprolactam and thus a higher overall yield to ε-caprolactam is obtained.

The process according to the invention is perfomed at a pressure of between about 0.5 and 20 Mpa.

It has been found that N-(5-carboxypentyl) ε-caprolactam has a beneficial effect on the rate of reaction for preparing ε-caprolactam from 6-aminocaproic acid, 6-aminocaproamide, 6-aminocaproic ester, 6-aminocapronitrile, oligomers or polymers of these compounds or mixtures comprising at least two of these compounds. The invention therefore also relates to the use of N-(5-carboxypentyl)-ε-caprolactam as reaction rate enhancing compound in a process for preparing ε-caprolactam from 6-aminocaproic acid, 6-aminocaproamide, 6-aminocaproic ester, 6-aminocapronitrile, oligomers or polymers of these compounds or mixtures comprising at least two of these compounds.

It has also been found that with the process according to the invention a composition is obtained comprising (a) ε-caprolactam and (b) N-(5-carboxypentyl)-s-caprolactam, wherein the amount of component (b) in the composition is lower than 50 wt % (based on the total composition).

The invention also relates to a process for the preparation of polyamide-6 starting from water and 6-aminocaproic acid, 6-aminocaproamide, 6-aminocaproic ester, 6-aminocapronitrile, and/or ε-caprolactam which process in performed in the presence of N-(5-carboxypentyl)-ε-caprolacatam and/or derivative thereof in an amount of less than 50 wt % and more than 0.1 wt %. A process for the hydrolytic polymerisation of ε-caprolactam into polyamide-6 is for example described in Kunststoff Handbuch ¾, Polyamide, Becker/Braun, Hanser Verlag, 1998, pages 41-47. A process for preparing polyamide-6 starting from 6-aminocapronitrile, and water is for example described in WO-A-9808889. It has been found that the presence of N-(5-carboxypentyl)-ε-caprolactam in such an amount has a beneficial effect on the rate of the hydrolytic polymerisation of 6-aminocaproic acid, 6-aminocaproamide, 6-aminocaproic ester, 6-aminocapronitrile and/or ε-caprolactam.

The invention will be elucidated by the following examples, however these are not intended to limit the scope of the invention in any way.

### Example I

A 500 ml autoclave having a turbine mixer was filled with 54 g 6-aminocaproic acid and 6.0 grams of N-(5-carboxypentyl)-ε-caprolactam. The autoclave was subsequently flushed with nitrogen and a pressure valve was adjusted such that the pressure in the reactor was maintained at 1.2 Mpa. When the temperature had reached 300 °C, the substrate feed, consisting of an aqueous 14.5 wt% 6-aminocaproic acid solution, was started at a rate of 260 ml per hour. At the reaction conditions of 300 °C and 1.2 MPa the water in the feed is momentaneously converted into steam, which strips *ε*-caprolactam from the reaction melt.
After 1 hour a steady state was reached. The experiment was continued for another 7 hours at steady state condition. The reactor contents at steady state conditions amounted to 50 grams of Nylon-6-melt, containing 12 wt% N-(5-carboxypentyl)-ε-caprolactam.
The steam containing ε-caprolactam leaving the reactor was condensed and analyzed. The accumulated condensed product, which was colourless on visual inspection, contained 254 grams of s-caprolactam, corresponding to 99% yield.
Kinetics were fit using the Arrhenius equation, in which the activation energy was fixed and the rate constant k₀ calculated at 2.41 ×10⁵ min⁻¹.

### Comparative Experiment A

Example I was repeated without the addition of N-(5-carboxypentyl)-ε-caprolactam, keeping all other reaction conditions identical to example 1. At steady state, the reactor contained 67 grams of nylon-6 melt, and k₀ was calculated at 1.80 ×10⁵ min⁻¹.

### Example 2

Example 1 was repeated, using reductive amination mixture as raw material, consisting of a mixture of ε-caprolactam (33.9 wt%) and ε-caprolactam precursors (10.9 wt% 6-aminocaproic acid, 38.3 wt% 6-aminocapronamide, 13.6 wt% nylon-6 oligomers), as well as some N-(5-carboxypentyl) -caprolactam (0.2 wt%) and water (3.1 wt%). Prior to reaction, 71 grams of reductive amination mixture was charged to the reactor, and after flushing the autoclave with nitrogen and heating to 300 °C at 1.2 MPa pressure, the feed of an aqueous reductive amination mixture with a concentration corresponding to 14.5 wt% ε-caprolactam was started at a rate of 260 ml per hour.
Steady state conditions were reached after 1 hour. k₀ amounted to 1.85 x10⁵ min⁻¹ at this point, with 76 grams of melt in the reactor containing 0.2 % N-(5-carboxypentyl)-ε-caprolactam.
After 29 hours running time, the feed rate was increased to 400 ml per hour. Reaction was continued for a total of 56 hours, after which the reactor contained 87 grams of nylon-6 melt in the new steady state, containing 5.3 % N-(5-carboxypentyl)-ε-caprolactam. The reaction rate gradually increased during the course of the reaction; at t = 56 hours, k₀ was calculated at 2.45 x10⁵ min⁻¹, showing a rate enhancement comparable to example 1. The accumulated condensed product contained 2600 grams of ε-caprolactam.

## Claims

1. A process for the preparation of ε-caprolactam starting from 6-aminocaproic acid, 6-aminocaproamide, 6-aminocaproic ester, 6-aminocapronitrile, oligomers or polymers of these compounds or mixtures comprising at least two of these compounds,
characterized in that, the process is performed
in the presence of N-(5-carboxypentyl)-ε-caprolactam and/or derivative thereof in an amount of less than 50 wt.% and more than 0.1 wt.% (based on the total reaction mixture).

2. Process according to claim 1, characterized in that, the starting mixture is an aqueous mixture containing 6-aminocaproic acid, 6-aminocaproamide, oligomers thereof and ε-caprolactam which aqueous mixture is obtained through reductive amination of 5-formylvaleric acid or its ester.

3. Process according to claim 1, characterized in that, the starting mixture is an aqueous mixture containing 6-aminocapronitrile.

4. Use of N-(5-carboxypentyl)-ε-caprolactam and/or derivative thereof as reaction rate enhancing
compound in a process for preparing ε-caprolactam from 6-aminocaproic acid, 6-aminocaproamide, 6-aminocaproic ester, 6-aminocapronitrile, oligomers or polymers of these compounds or mixtures comprising at least two of these compounds.

5. A composition comprising (a) ε-caprolactam and (b)
N-(5-carboxypentyl)-ε-caprolactam, wherein the amount of component (b) in the composition is lower than 50 wt % (based on the total composition).

6. A process for the preparation of polyamide-6 starting from water and 6-aminocaproic acid, 6-aminocaproamide, 6-aminocaproic ester, 6-aminocapronitrile, and/or ε-caprolactam,
characterized in that, the process is performed in the presence of N-(5-carboxypentyl)-ε-caprolactam and/or derivative thereof in an amount of less than 50 wt % and more than 0.1 wt %.

7. Use of N-(5-carboxypentyl)-ε-caprolactam and/or derivative therof as reaction rate enhancing additive in a process for the preparation of polyamide-6 starting from water and ε-caprolactam, 6-aminocaproic acid, 6-aminocaproamide, 6-aminocaproic ester and/or 6-aminocapronitrile.
